# EUROPEAN PATENT APPLICATION

(11) **EP 1 757 271 A1**
(43) Date of publication of application: **28.02.2007**
(21) Application number: 05728538.9
(22) Date of filing: 08.04.2005
(51) Int. Cl.: A61K 9/14, A61K 9/16, A61K 9/28, A61K 47/02, A61K 47/36, A61K 47/38

(54) **SOLID PHARMACEUTICAL PREPARATION WITH IMPROVED STABILITY AND PROCESS FOR PRODUCING THE SAME**

(30) Priority: 08.04.2004 JP 2004114073
(71) Applicant: KYOWA HAKKO KOGYO CO., LTD., Chiyoda-ku, Tokyo 100-8185 (JP)
(72) Inventor: SHICHIBE, Shozo c/oKYOWA KAKKO KOGYO CO.,LTD, Nagoya-shi, Aichi 460-0002 (JP); ISHIKAWA, Yasuhiro c/o KYOWA HAKKO KOGYO CO.,LTD.,, Sunto-gun, Shizuoka 411-8731 (JP); KATO, Yasuki, Shizuoka 410-1115 (JP)
(74) Representative: Casalonga, Axel
(86) International application number: PCT/JP2005/006962
(87) International publication number: WO 2005/097070

(57) **Abstract**

An object of the present invention is to provide solid products excellent in storage stability such as photostability and a method for producing the solid products. The solid products comprising: intermediate products including a core part comprising an active substance, and a core-coating layer covering the core part and comprising a powder-coating agent; and a film covering the intermediate products and comprising a compound having a photocatalytic activity or a semiconducting property are provided. The method for producing the solid products which comprises the step of coating a core part comprising an active substance with constituent components of a core-coating layer comprising a powder-coating agent by a powder coating method is provided.

## Description

### Technical Field

The present invention relates to solid products excellent in storage stability such as photostability and a method for producing the same.

### Background Art

Solid products comprising an active substance such as medical drug products, food products, agricultural products and animal drug products are desired to be stable in an environment where they are used or stored. Solid products may be unstable to light in some cases depending on the active substance comprised in the solid products, and heretofore, many stabilization methods which comprise combining intermediate products comprising an active substance with a film comprising a light-resistant agent such as titanium oxide thereby to form products have been proposed. For example, solid products (fine granules, granules or a tablet) comprising intermediate products comprising sertindole and a film comprising titanium oxide as a light-resistant agent is known (see Patent document 1).

On the other hand, for example, titanium oxide or the like is a metal salt, therefore, when it comes into touch with an active substance, a chemical reaction may be induced in some cases. As the measures, a method of preventing an active substance from coming into touch with a film comprising titanium oxide or the like by covering the surface of intermediate products with an inactive substance is known. For example, solid products comprising a core part comprising vitamin K, a film comprising titanium oxide as a light-resistant agent and an in-between film comprising a water-soluble polymer between the core part comprising an active substance and the film are known (see Patent document 2).

Further, it is known that for example titanium oxide or the like generates free radical by ultraviolet light, and a chemical reaction of an active substance may be induced by free radical or a secondary substance generated by a reaction thereof in some cases. Accordingly, for example, medical drug products comprising intermediate products comprising an active substance and a film comprising a light-resistant agent that can generate free radical by ultraviolet light and a free radical scavenger have been reported (see Patent document 3). However, there is a concern that a free radical scavenger as it is or a substance generated by a reaction thereof with free radical or the like may make the active substance unstable.
Patent document 1: International Publication No. WO 97/39752
Patent document 2: JP-A-2002-104960
Patent document 3: JP-A-11-147819

### Disclosure of the invention

### Problems to be Solved by the Invention

An object of the present invention is to provide solid products, which comprises intermediate products comprising an active substance and a film covering the intermediate products and are excellent in storage stability such as photostability, and a method for producing the same.

### Means for Solving the Problems

The present invention relates to the following (1) to (9).
(1) Solid products comprising: intermediate products including a core part comprising an active substance, and a core-coating layer covering the core part and comprising a powder-coating agent; and a film covering the intermediate products and comprising a compound having a photocatalytic activity or a semiconducting property.
(2) The solid products according to the above (1), wherein the compound having a photocatalytic activity or a semiconducting property is titanium oxide, iron oxide, zinc oxide or silicon oxide.
(3) The solid products according to the above (1) or (2), wherein the active substance is an active substance having reactivity with free radical or a secondary substance generated by a reaction thereof.
(4) The solid products according to any one of the above (1) to (3), wherein the powder-coating agent is one or more member(s) selected from sugars, starches, starch derivatives, celluloses and cellulose derivatives.
(5) The solid products according to any one of the above (1) to (3), wherein the powder-coating agent is a combination of a sugar, a starch and/or a slightly water-soluble inorganic salt.
(6) The solid products according to the above (4) or (5), wherein the sugar is lactose or sucrose.
(7) The solid products according to any one of the above (1) to (6, wherein the form of the solid products are powders, fine granules or granules.
(8) The solid products according to any one of the above (1) to (6), wherein the form of the solid products are tablets.
(9) A method for producing the solid products according to any one of the above (1) to (8), which comprises the step of coating a core part comprising an active substance with constituent components of a core-coating layer comprising a powder-coating agent by a powder coating method.

### Effect of the Invention

According to the present invention, solid products excellent in storage stability such as photostability and a method for producing the same are provided.

### Best Mode for Carrying Out the Invention

Solid products of the present invention are solid products comprising intermediate products comprising an active substance and a film covering the intermediate products. The intermediate products in the solid products of the present invention are products that can be coated. The form of the intermediate products are not particularly limited, however, these are preferably in the form of powders, fine granules, granules or tablets. In this description, intermediate products in the form of powders, fine granules or granules are referred to as intermediate granules, and intermediate products in the form of tablets are referred to as intermediate tablets. The intermediate products in the solid products of the present invention comprise a core part comprising an active substance and a core-coating layer covering the core part and comprising a powder-coating agent. The core part is products that can be powder coated, and the core-coating layer is a coating layer covering the core part. Further, the film in the solid products of the present invention is a film further covering the outside of the core-coating layer, and for example, a film formed by coating with a coating composition comprising a compound having a photocatalytic activity or a semiconducting property.

The active substance in the present invention is not particularly limited, however, preferred examples thereof include an active substance having reactivity with free radical generated by a compound having a photocatalytic activity or a semiconducting property or a secondary substance generated by a reaction thereof, such as superoxide radical (O₂⁻, O₂H) or hydroxyl radical (OH) and the like. The active substance having reactivity with free radical or a secondary substance generated by a reaction thereof includes those having direct or indirect reactivity with free radical, and examples thereof include an active substance degraded by superoxide radical or hydroxyl radical, an active substance degraded by an aldehyde (such as formaldehyde or acetaldehyde), an acid (such as formic acid), a peroxide or the like generated by reacting superoxide radical or hydroxyl radical with a component in products and the like. More specific examples include vitamin A, vitamin D, vitamin E, vitamin B₁, vitamin B₂, vitamin B₆, vitamin C, vitamin B₁₂, proteins, amino acids, oligosaccharides, aspirin, acetaminophen, ethenzamide, ibuprofen, diphenhydramine, chlorpheniramine, dihydrocodeine, noscapine, methylephedrine, caffeine, serrapeptase, lysozyme, diclofenac sodium, ketoprofen, indomethacin, bucolome, pentazocine, chlorpromazine, reserpine, alprazolam, chlordiazepoxide, diazepam, imipramine, maprotiline, estazolam, nitrazepam, diazepam, phenobarbital sodium, scopolamine, papaverine, citicoline, meclofenoxate, phenytoin, carbamazepine, isoproterenol, diastase, resistant lactic acid bacteria, bifidobacteria, lansoprazole, omeprazole, rabeprazole, famotidine, cimetidine, ranitidine, dextromethorphan, guanfacine, codeine, difenidol, metoclopramide, levallorphan, theophylline, salbutamol, amlexanox, seratrodast, oxytetracycline, triamcinolone acetonide, chlorhexidine, lidocaine, diphenhydramine, promethazine, isothipendyl, digoxin, procainamide, propranolol, pindolol, isosorbide, furosemide, delapril, captopril, hydralazine, labetalol, manidipine, candesartan cilexetil, methyldopa, losartan, valsartan, eprosartan, irbesartan, tasosartan, telmisartan, forasartan, phenylephrine, carbocromen, molsidomine, verapamil, simvastatin, pravastatin, trepibutone, cefalexin, amoxicillin, pivmecillinam, cefotiam, cefozopran, cefmenoxime, cefsluodin sodium, ampicillin, cyclacillin, sulbenicillin sodium, nalidixic acid, enoxacin, carumonam sodium, tolbutamide, voglibose, pioglitazone, troglitazone, acarbose, miglitol, emiglitate, ipriflavone, methocarbamol, meclizine, dimenhydrinate, liothyronine sodium, dexamethasone, prednisolone, oxendolone, leupororelin, opium, morphine, ipecac, oxycodone, opium alkaloids, cocaine, allopurinol, colchicine, 5-fluorouracil, mitomycin, dibenz[b,e]oxepin derivatives (hereinafter referred to as compound (I)) represented by the formula (I):

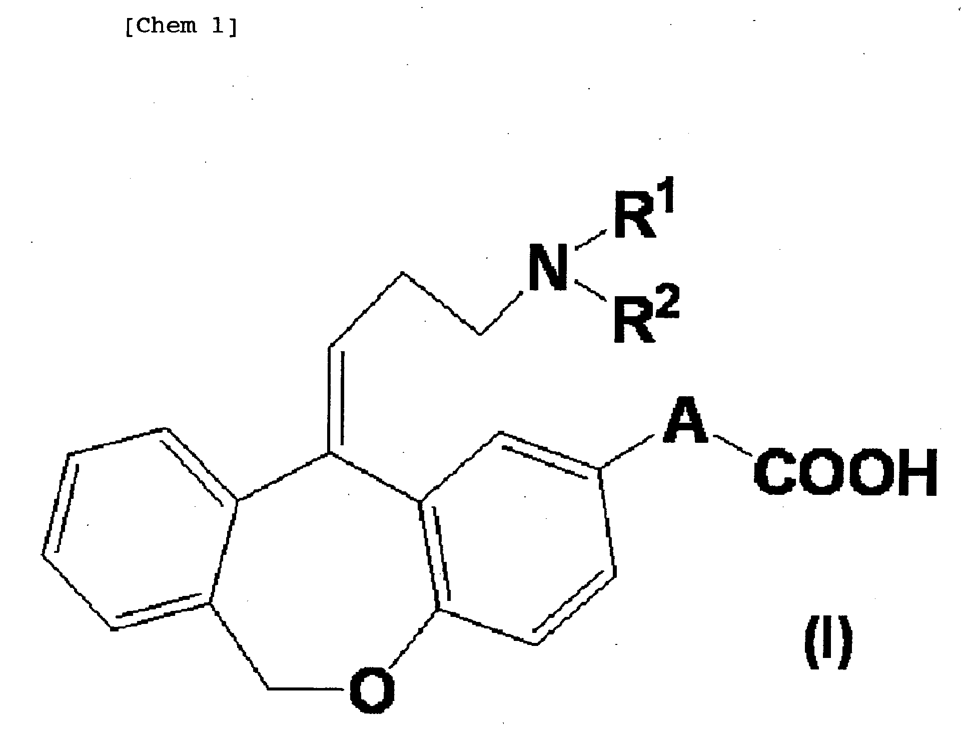

[wherein A represents a single bond, -CH=CH- or (CH₂)ₙ-(wherein n represents an integer of 1 to 3), R¹ and R² are the same or different and represent hydrogen or lower alkyl, or are combined together with the adjacent nitrogen atom to form a heterocyclic group], pharmaceutically acceptable salts thereof and the like. In particular, an active substance having an amino group or an imino group is preferred.

The pharmaceutically acceptable salt includes, for example, a pharmaceutically acceptable acid addition salt, metal salt, ammonium salt, organic amine addition salt, amino acid addition salt and the like. Examples of the pharmaceutically acceptable acid addition salt include inorganic acid salts such as a hydrochlorate, a hydrobromate, a nitrate, a sulfate and a phosphate, and organic acid salts such as an acetate, a maleate, a fumarate, a tartarate and a citrate. Examples of the pharmaceutically acceptable metal salt include alkali metal salts such as a lithium salt, a sodium salt and a potassium salt, alkaline earth metal salts such as a magnesium salt and a calcium salt, an aluminum salt, a zinc salt and the like. Examples of the pharmaceutically acceptable ammonium salt include salts of ammonium, tetramethylammonium and the like. Examples of the pharmaceutically acceptable organic amine addition salt include addition salts of morpholine, piperidine and the like. Examples of the pharmaceutically acceptable amino acid addition salt include addition salts of lysine, glycine, phenylalanine, aspartic acid, glutamic acid and the like.

In the definition of each group of the formula (I), as the lower alkyl, for example, linear or branched alkyl having 1 to 6 carbon atoms can be exemplified, and specific examples thereof include methyl, ethyl, propyl, isopropyl, butyl, isobutyl, sec-butyl, tert-butyl, pentyl, hexyl and the like. Examples of the heterocyclic group formed by combining with the adjacent nitrogen atom include pyrrolidinyl, morpholino, thiomorpholino, N-methylpiperazinyl, pyrazolidinyl, piperidino, piperazinyl, indolyl, isoindolyl and the like.

The compound (I) can be produced by or in accordance with the method disclosed in JP-A-63-10784.

Among the compound (I), a compound in which A is CH₂, and both R¹ and R² are CH₃ is preferred, and as a preferred specific example of the compound (I) or a pharmaceutically acceptable salt thereof, (Z)-11-(3-dimethylaminopropylidene) -6,11-dihydrodibenz[b,e]oxepin-2-acetic acid hydrochloride (hereinafter referred to as Compound (A)) represented by the formula (A):

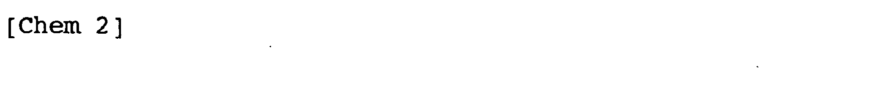

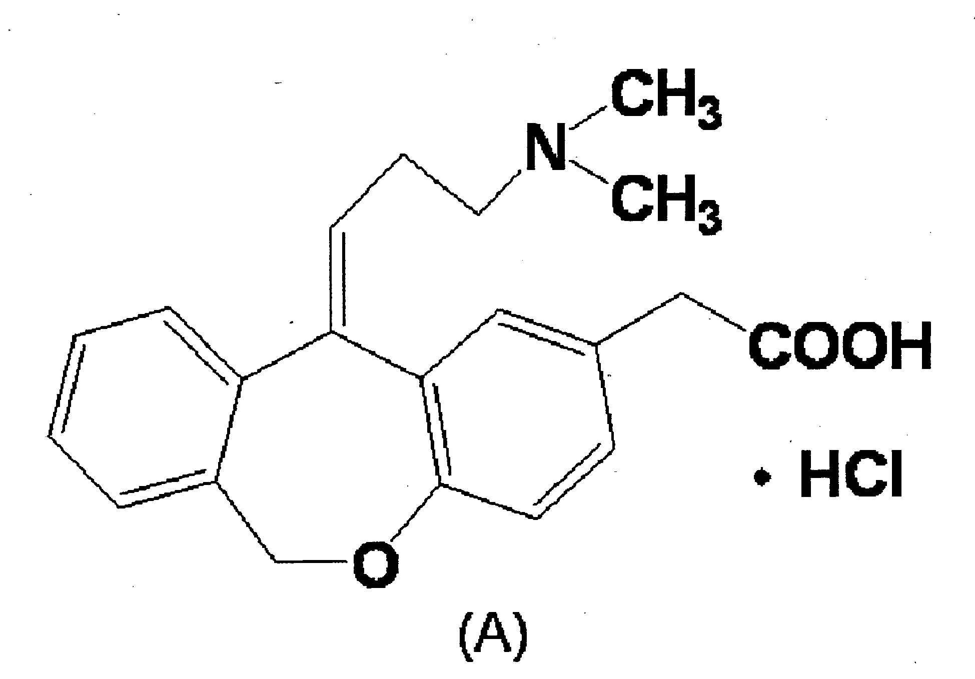

can be exemplified.

The powder-coating agent in the present invention is a solid excipient or one obtained by mixing a solid excipient with a liquid or semisolid excipient, and the solid excipient is a powder or a crystalline powder. Further, in the production of the solid products of the present invention, the particle size of the powder-coating agent to be used is preferably about 100 µm or less, more preferably about 50 µm or less, further more preferably about 40 µm or less in terms of a volume average particle size measured by microscope or a sieving method. This is also preferred in the production of intermediate products from the viewpoint of obtaining the intermediate products in high yield.

Examples of the excipient as the powder-coating agent include sugars (such as lactose, sucrose and maltose), sugar alcohols (such as mannitol, maltitol and erythritol), starches (such as corn starch, rice starch and wheat starch), starch derivatives (such as hydroxypropyl starch and sodium carboxymethyl starch), celluloses (such as crystalline cellulose and powdered cellulose), cellulose derivatives (such as methylcellulose, carboxypropylcellulose, hydroxypropylmethylcellulose, croscarmellose sodium, low substituted hydroxypropylcellulose, carboxymethylcellulose and calcium carboxymethylcellulose), slightly water-soluble inorganic salts (such as talc, light anhydrous silicic acid, sodium aluminometasilicate and calcium phosphate) and the like, and these may be used alone or in combination of two or more of them. Preferred examples include one or more substances selected from sugars, starches, starch derivatives, celluloses, cellulose derivatives and the like (specifically, sucrose or lactose among sugars, corn starch among starches, hydroxypropyl starch or sodium carboxymethyl starch among starch derivatives, crystalline cellulose among celluloses, croscarmellose sodium, low substituted hydroxypropylcellulose, carboxymethylcellulose or calcium carboxymethylcellulose among cellulose derivatives and the like), more preferred examples include one or more substances selected from sugars, starches, celluloses and the like (specifically, lactose, sucrose, corn starch, crystalline cellulose and the like), and the most preferred examples include sugars (specifically, sucrose and the like). More preferably, the excipient is selected from excipients having an equilibrium moisture content of preferably 10% or less, more preferably 5% or less. In the case where the excipients are used in combination, preferably, one or more substances selected from sugars, starches, starch derivatives, celluloses, cellulose derivatives and the like (specifically, sucrose or lactose among sugars, corn starch among starches, hydroxypropyl starch or sodium carboxymethyl starch among starch derivatives, crystalline cellulose among celluloses, croscarmellose sodium, low substituted hydroxypropylcellulose, carboxymethylcellulose or calcium carboxymethylcellulose among cellulose derivatives and the like) are mainly used, and more preferably, one or more substances selected from sugars, starches, celluloses and the like (specifically, lactose, sucrose, corn starch, crystalline cellulose and the like) are mainly used, and most preferably, a sugar (specifically, sucrose or the like) is mainly used. In the case where a sugar is mainly used, preferably a starch and/or a slightly water-soluble inorganic salt are/is used in combination. In the case where lactose or sucrose is mainly used, more preferably corn starch, talc or light anhydrous silicic acid is used in combination. The use of a starch and/or a slightly water-soluble inorganic salt in combination in the case where a sugar is mainly used, is also preferred in the production of intermediate products in terms of obtaining the intermediate products in high yield.

The intermediate products in the solid products of the present invention comprise a core part comprising an active substance, and a core-coating layer comprising a powder-coating agent, and the core part may comprise another active substance and/or an excipient (the same definition as the above-mentioned excipient) in addition to the active substance.

Examples of the excipient comprised in the core part comprise a diluent, a disintegrator, a binder, a lubricant and the like, and a combination thereof.
Examples of the diluent include sugars (such as lactose, sucrose and maltose), sugar alcohols (such as mannitol, maltitol and erythritol), starches (such as corn starch, rice starch and wheat starch), celluloses (such as crystalline cellulose and powdered cellulose), cellulose derivatives (such as croscarmellose sodium, low substituted hydroxypropylcellulose, carboxymethylcellulose and calcium carboxymethylcellulose), slightly water-soluble inorganic salts (such as talc, light anhydrous silicic acid, sodium aluminometasilicate and calcium phosphate) and the like, and these are used alone or in combination of two or more of them. Preferred examples include one or more substances selected from sugars, starches, celluloses, cellulose derivatives and the like (specifically, sucrose or lactose among sugars, corn starch among starches, crystalline cellulose among celluloses, croscarmellose sodium, low substituted hydroxypropylcellulose, carboxymethylcellulose or calcium carboxymethylcellulose among cellulose derivatives and the like), and more preferred examples include one or more substances selected from sugars, starches, celluloses and the like (specifically, lactose, sucrose, corn starch, crystalline cellulose and the like).

Examples of the disintegrator include celluloses (such as crystalline cellulose and powdered cellulose), cellulose derivatives (such as croscarmellose sodium, low substituted hydroxypropylcellulose and carmellose calcium), starches (such as corn starch, pregelatinized starch and partly pregelatinized starch), starch derivatives (such as hydroxypropyl starch and sodium carboxymethyl starch), crospovidone, bentonite and the like, and these may be used alone or in combination of two or more of them.

Examples of the binder include cellulose derivatives (such as methylcellulose, carboxymethylcellulose, carboxypropylcellulose, hydroxypropylcellulose and hydroxypropylmethylcellulose), celluloses (such as crystalline cellulose), starches (such as pregelatinized starch), polyvinyl alcohol, polyvinylpyrrolidone, pullulan, dextrin, acacia, gelatin and the like, and these may be used alone or in combination of two or more of them.

Examples of the lubricant include magnesium stearate, calcium stearate, hydrogenated oil, sucrose esters of fatty acid, polyethyleneglycol and the like, and these may be used alone or in combination of two or more of them.

Further, the core-coating layer may comprise an excipient (such as a binder (the same definition as the binder in the above-mentioned core part) or a lubricant (the same definition as the lubricant in the above-mentioned core part) in addition to the powder-coating agent as long as it does not inhibit the coating of the above-mentioned core part with the constituent components of the core-coating layer including the powder-coating agent in the production process, and may comprise the above-mentioned active substance as desired.

The film in the solid products of the present invention comprises a compound having a photocatalytic activity or a semiconducting property. Examples of the compound (hereinafter referred to as Compound B) include metal compounds, silicon compounds, organic compounds, complexes and the like having a photocatalytic activity or a semiconducting property, preferred examples include metal compounds, silicon compounds and the like having a photocatalytic activity or a semiconducting property, more preferred examples include titanium oxide, iron oxide (specifically, yellow ferric oxide, red ferric oxide, yellow oxide of iron, black iron oxide and the like), zinc oxide, silicon oxide and the like, and further more preferred examples include titanium oxide. The titanium oxide in the present invention may be in either crystal form of a rutile type or an anatase type, and also the particle size thereof is not particularly limited. As for such titanium oxides, two or more types having different crystal forms or particle sizes may be used in combination. Further, as for Compound B, preferably two or more types having different colors are used in combination. The combination in the case where two or more types of Compounds B are used in combination is not particularly limited, however, preferred examples include a combination of titanium oxide with iron oxide (specifically, yellow ferric oxide, red ferric oxide, yellow oxide of iron, black iron oxide or the like), zinc oxide, silicon oxide or the like, and more preferred examples include a combination of titanium oxide with iron oxide (specifically, yellow ferric oxide, red ferric oxide or the like).

The film in the solid products of the present invention may comprise other light-resistant agent, a lubricant or a dispersant in addition to the above-mentioned Compound B. For example, bengara, carbon black, medicinal carbon, barium sulfate, food yellow No. 4 aluminum lake, food red No. 2, food red No. 3, food red No. 102, copper chlorophene and the like can be exemplified.

The film in the solid products of the present invention may further comprise a coating agent such as a coating base agent, a plasticizer or a binder, and such a coating agent may be used as a mixture of two or more types, respectively.

Examples of the coating base agent include a gastric film-coating agent, an enteric film-coating agent, a sustained release-film coating agent and the like. Examples of the gastric film-coating base agent include cellulose polymers such as hydroxypropylcellulose, hydroxypropylmethylcellulose, hydroxyethylcellulose and methylhydroxyethylcellulose, synthetic polymers such as polyvinylacetal diethylaminoacetate, aminoalkyl methacrylate copolymer E and polyvinylpyrrolidone, polysaccharides such as pullulan and the like. Examples of the enteric film-coating base agent include cellulose polymers such as hydroxypropylmethylcellulose phthalate, hydroxypropylmethylcellulose acetate succinate, carboxymethylethylcellulose and cellulose acetate phthalate, acrylic acid polymers such as methacrylic acid copolymer L, methacrylic acid copolymer LD and methacrylic acid copolymer S, natural compounds such as shellac and the like. Examples of the sustained release-film coating base agent include cellulose polymers such as ethylcellulose, acrylic acid polymers such as aminoalkyl methacrylate copolymer RS and ethyl acrylate methyl methacrylate copolymers and the like.

Examples of the plasticizer include esters such as triethyl citrate, medium chain fatty acid triglyceride, diethyl phthalate, dibutyl phthalate, triacetine, butylphthalyl butylglycolate and glyceryl caprylate, alcohols such as glycerin, propylene glycol and polyethylene glycol and the like.

Examples of the binder and other coating agents include lactose, sucrose, talc, calcium carbonate, gelatin, acacia, carnauba wax and the like.

The core part in the solid products of the present invention may be of any shape such as spherical shape, cylindrical shape, indefinite shape or the like. The particle size is an arbitrary particle size to be generally used (for example, 0.4 to about 2.0 mm in terms of a volume average particle size measured by microscope or a sieving method). Further, the core part may be composed of a lead part and a lead-coating layer. The core part can be produced by, for example, a wet granulation method, a dry granulation method or the like. Examples of the wet granulation method include an extrusion granulation method (with a screw extrusion granulator, a roll extrusion granulator or the like), a tumbling granulation method (with a rotary drum granulator, a centrifugal tumbling granulator or the like), a fluidized bed granulation method (with a fluidized bed granulator, a tumbling fluidized bed granulator or the like), a stirring granulation method (with a stirring granulator or the like) and the like. More specifically, for example, a method in which an active substance and an excipient are mixed, a solvent or a binder solution is added to the resulting mixture followed by granulation, then the resulting granulated products are dried and the like can be exemplified. Examples of the solvent include water, ethanol, isopropyl alcohol, a solvent mixture thereof and the like. Examples of the binder solution include those obtained by dissolving a binder in water, ethanol, isopropyl alcohol, a solvent mixture thereof and the like, however, the most suitable is an aqueous solution of a binder. As the dry granulation method, for example, a pulverizing granulation method in which flakes are produced using a commercially available dry granulator or slug tablets are produced with a tableting machine, and the resulting flakes or slug tablets are pulverized with a commercially available crusher or pulverizer to produce granulated products and the like can be exemplified. Further, preferably, the respective granulated products are suitably ground and/or sieved so as to obtain products with a desired particle size.

On the other hand, in the case where the core part is composed of a lead part and a lead-coating layer, the lead part is produced in the same manner as the production of the above-mentioned core part (it is preferred that the lead part does not comprise an active substance), or can be obtained as a commercially available spherical seed particles. The lead-coating layer can be produced by coating the lead part with components of the lead-coating layer (including an active substance) by a powder coating method or a spray coating method, and these are preferably produced by a powder coating method. The production of the lead-coating layer by a powder coating method can be carried out by coating the lead part with components of the lead-coating layer in the same manner as the case of coating with constituent components of the core-coating layer including a powder-coating agent in the step of producing a intermediate granules having the core-coating layer in the process for producing granules described below. Preferred embodiments of the core-coating layer are also preferred embodiments of the lead-coating layer, respectively. The production of the lead-coating layer by a spray coating method is carried out by dissolving or suspending an active substance and an excipient as needed in a solvent (the same definition as the above-mentioned solvent) or a binder solution (the same definition as the above-mentioned binder solution), and spraying the mixture over the lead part. The spray coating method is carried out using, for example, a conventional pan coating machine, vented coating machine, fluidized bed coating machine, tumbling fluidized coating machine or the like. The spherical seed particles are composed of, for example, purified sucrose, a mixture of sucrose and corn starch, a mixture of lactose and crystalline cellulose, crystalline cellulose or the like. The particle size of the seed particles is generally 180 to 1180 µm in terms of a volume average particle size measured by microscope or a sieving method.

The solid products of the present invention can be produced by, for example, a production method comprising the steps of producing intermediate products by coating the above-mentioned core part with a powder-coating agent, and as desired, carrying out spheronization, pulverization and/or tableting (together with another excipient as desired); and producing solid products having a film by coating the resulting intermediate products with a coating composition comprising Compound B.

Hereinafter, the method for producing the solid products of the present invention will be described in detail for the respective forms. However, the following production methods are provided only for illustrations and not intended to limit the process for producing the solid products of the present invention.

### [Method for producing granules]

Among the solid products of the present invention, solid products in the form of granules are produced by, for example, a production method comprising the steps of producing intermediate granules having a core-coating layer by coating the above-mentioned core part with the above-mentioned powder-coating agent, and producing a granules having a film by coating the intermediate granules with a coating composition comprising the above-mentioned Compound B.

The intermediate granules having a core-coating layer can be produced by, for example, coating a core part with constituent components of a core-coating layer including a powder-coating agent (it is preferred that they do not comprise an active substance) by a powder coating method. The powder coating method is a method in which an excipient and as needed, an active substance are mixed to prepare a powder-coating agent, and the powder-coating agent is dispersed while a solvent (the same definition as the above-mentioned solvent) or a binder solution (the same definition as the above-mentioned binder solution) is sprayed, whereby the powder-coating agent is laminated onto a core part. The powder coating method is carried out using, for example, a rotary drum granulator, a centrifugal tumbling granulator, a tumbling granulator, a tumbling fluidized bed granulator or the like. The core-coating layer is not limited to one layer, and may be formed with a plurality of layers. In this case, the types and/or the blending amounts of the components to be used for the respective layers may be changed.

The core part may be of any shape such as spherical shape, cylindrical shape, indefinite shape or the like, however, a spherical shape is preferred so as to easily form the core-coating layer and the film uniformly. In light of this, a core part produced by kneading a mixture of an active substance and an excipient (with a stirring granulator or the like) followed by extrusion granulation (with a screw extrusion granulator, a roll extrusion granulator or the like) and then spheronization (with a rotating disk granulator, a centrifugal tumbling granulator, a tumbling fluidized bed granulator or the like), or a core part produced by using a spherical seed particles as a lead part and coating the lead part with components of a lead-coating layer (including an active substance) are more preferred. The particle size of the core part is preferably 180 to 1180 µm, more preferably 300 to 650 µm in terms of a volume average particle size measured by microscope or a sieving method.

The granules having a film can be produced by, for example, dissolving and/or dispersing a coating composition comprising Compound B in a solvent (the same definition as the above-mentioned solvent), and coating a intermediate granules produced as described above with the coating composition. The coating is carried out using, for example, a conventional pan coating machine, vented coating machine, fluidized bed coating machine, tumbling fluidized coating machine, centrifugal tumbling coating machine or the like.

### [Method for producing powders or fine granules]

Among the solid products of the present invention, solid products in the form of powders may be any as long as these are general powders, and for example, powders that does not pass through a No. 30 sieve (500 µm) in an amount of 5% or less of the total amount of the powders are preferred.

Among the solid products of the present invention, solid products in the form of a fine granules may be any as long as these are general fine granules, and for example, fine granules that does not pass through a No. 30 sieve (500 µm) in an amount of 5% or less of the total amount of the powders and that passes through a No. 200 sieve (75 µm) in an amount of 10% or less of the total amount of the powders are preferred.

Among the solid products of the present invention, solid products in the form of powders or a fine granules can be produced, for example, by producing granules in the same manner as the above-mentioned method for producing granules, and then appropriately pulverizing and/or sieving these in the respective steps to give a products with a desired particle size.

### [Method for producing tablets]

Among the solid products of the present invention, solid products in the form of a tablet can be produced by, for example, a production method comprising the steps of adding powder and/or granulated products of an excipient (the same definition as the excipient in the above-mentioned core part) as needed to intermediate granules having a core-coating layer in the method for producing the above-mentioned granules, powders or fine granules and mixing them, producing intermediate tablets by tableting the resulting mixture, and producing tablets having a film by coating the intermediate tablet with a coating composition comprising Compound B.

The production of the granulated products can be carried out by, for example, a wet granulation method, a dry granulation method or the like. Examples of the wet granulation method include an extrusion granulation method (with a screw extrusion granulator, a roll extrusion granulator or the like), a tumbling granulation method (with a rotary drum granulator, a centrifugal tumbling granulator or the like), a fluidized bed granulation method (with a fluidized bed granulator, a tumbling fluidized bed granulator or the like), a stirring granulation method (with a stirring granulator or the like) and the like. More specifically, for example, a method in which an excipient is mixed, a solvent (the same definition as the above-mentioned solvent) or a binder solution (the same definition as the above-mentioned binder solution) is added to the resulting mixture followed by granulation, and then the resulting granulated products are dried and the like can be exemplified. As the dry granulation method, for example, a pulverizing granulation method in which flakes are produced using a commercially available dry granulator or slug tablets are produced with a tableting machine, and the resulting flakes or slug tablets are pulverized with a commercially available crusher or pulverizer to obtain granulated products and the like can be exemplified. Further, preferably, the respective granulated products are suitably ground and/or sieved so as to obtain products with a desired particle size.

The intermediate tablet can be produced by adding powder and/or granulated products of an excipient as needed to an intermediate granules having a core-coating layer and mixing them, and tableting the resulting mixture as described above, and the tableting pressure upon tableting can be appropriately selected, for example, in the range of 3 to 30 kN. The weight of the intermediate tablet is not particularly limited, however, it is, for example, 30 to 3000 mg per one tablet.

The tablet having a film can be produced by, for example, dissolving and/or dispersing a coating composition comprising Compound B in a solvent (the same definition as the above-mentioned solvent) and coating the above-mentioned intermediate tablet with the coating composition. The coating is carried out using, for example, a conventional pan coating machine, vented coating machine, fluidized bed coating machine, tumbling fluidized coating machine or the like.

The solid products of the present invention may be in any form as long as it can be orally administered, however, in particular, these are preferably in the form of powders, a fine granules, a granules or a tablet, and more preferably in the form of fine granules or granules. Further, the solid products of the present invention may be packed in capsules to form capsules comprising the solid products of the present invention, or it may be mixed with another excipient or the like as needed and/or the mixture is granulated to prepare particle products or dry syrups comprising the solid products of the present invention or to prepare multiple unit tablets comprising the solid products of the present invention.

### [Method for producing multiple unit tablets]

The multiple unit tablet comprising the solid products of the present invention can be produced by a production method comprising the steps of adding powder and/or granulated products (the same definition as the granulated products in the production of a intermediate tablet in the solid products of the present invention in the form of a tablet) of an excipient (the same definition as the excipient in the above-mentioned core part) as needed to the solid products of the present invention and mixing them, and tableting the resulting mixture in the same manner as the production of a intermediate tablet in the solid products of the present invention in the form of a tablet. At this time, tableting is preferably carried out by an external lubrication tableting method. Further, a multiple unit tablet rapidly disintegrating in the oral cavity can be produced by a production method comprising the step of mixing the solid products of the present invention which are fine granules when a volume average particle size measured by microscope or a sieving method is 400 µm or less with a sugar alcohol (such as mannitol, maltitol or erythritol) a disintegrator (the same definition as the disintegrator in the above-mentioned core part) and if necessary another excipient (the same definition as the excipient in the above-mentioned core part).

In the film in the solid products of the present invention, the amount of Compound B in the film is preferably 0.6 part by weight or more, more preferably 0.8 to 50 parts by weight, further more preferably 1 to 25 parts by weight relative to 100 parts by weight of the intermediate products. Further, in the case where titanium oxide or a combination of titanium oxide and iron oxide (specifically, yellow ferric oxide, red ferric oxide or the like) was used as Compound B, the amount of titanium oxide or both titanium oxide and iron oxide is preferably 0.6 part by weight or more, more preferably 0.8 to 50 parts by weight, further more preferably 1 to 25 parts by weight relative to 100 parts by weight of the intermediate products.

The amount of the film relative to the intermediate products are preferably 1 to 1000 parts by weight, more preferably 1.5 to 100 parts by weight, further more preferably 1.5 to 50 parts by weight relative to 100 parts by weight of the intermediate products. When a coating composition comprising Compound B for coating intermediate products are dissolved and/or dispersed in a solvent, generally the concentration of the coating composition is preferably 0.1 to 50 % by weight, more preferably 0.5 to 20% by weight.

As for the active substance in the intermediate products in the solid products of the present invention, the amount of the active substance in the core-coating layer is preferably 0.5 parts by weight or less relative to 100 parts by weight of the total components in the core-coating layer. More preferably, the amount of the active substance in the core-coating layer is preferably 0.05 to 0.5 parts by weight relative to 100 parts by weight of the total components in the core-coating layer and the remaining active substance is comprised in the core part, or the active substance is not comprised in the core-coating layer and all the active substance is comprised in the core part. Further more preferably, the active substance is not comprised in the core-coating layer and all the active substance is comprised in the core part. As described above, it is preferred that in the intermediate products in the solid products of the present invention, the active substance is not comprised in the core-coating layer and all the active substance is comprised in the core part. In this case, the decrease in stability of the active substance due to the touch with the components in the film, or air or humidity outside the products or the loss of active substance due to migration thereof to the outside of the products can be further suppressed.

The amount of the respective excipients comprised in the solid products of the present invention may be within the range of the amount generally used. Further, in the case where a binder is used as a binder solution, generally the concentration of the binder in the binder solution is preferably 0.1 to 50% by weight, more preferably 0.5 to 20% by weight. The solid products of the present invention may comprise a binder added in the form of a powder in addition to the binder added by spraying the binder solution.

Further, the amount of the core-coating layer is preferably 5 to 90 parts by weight, more preferably 10 to 75 parts by weight, further more preferably 10 to 50 parts by weight relative to 100 parts by weight of the intermediate products. When the amount of the core-coating layer is 5 parts by weight or more, it becomes more easy to perform coating of the entire core part with a powder-coating agent, and when it is 90 parts by weight or less, it becomes more easy to produce intermediate products with a particle size suitable as fine granules or granules.

In the case where the core part is composed of a lead part and a lead-coating layer, the amount of the lead-coating layer is preferably 5 to 90 parts by weight, more preferably 10 to 75 parts by weight, further more preferably 10 to 50 parts by weight relative to 100 parts by weight of the lead part when performing the production of the lead-coating layer by a powder coating method. When performing the production of the lead-coating layer by a spray coating method, the amount of the lead-coating layer is preferably 0.1 to 90 parts by weight, more preferably 0.5 to 50 parts by weight, further more preferably 1 to 30 parts by weight relative to 100 parts by weight of the lead part.

Hereinafter, the present invention will be described in more detail with reference to Examples, however, the invention is not limited to these Examples.

### Example 1

Granules shown in Table 1 were produced in accordance with the following procedure.
Intermediate granules: A powder mixture of 6 g of Compound (A) (manufactured by Kyowa Hakko Co. Ltd., the same applies hereinafter) and 114 g of sucrose powder (Japanese Pharmacopoeia sucrose, manufactured by Nissin Sugar Manufacturing Co. Ltd., the same applies hereinafter) were dispersed onto 600 g of purified sucrose spherical granules (Nonpareil-103, 32-42 mesh, manufactured by Freund Industrial Co. Ltd., the same applies hereinafter) as a lead part using a centrifugal tumbling granulator (CF-360; CF granulator 360, manufactured by Freund Industrial Co. Ltd., the same applies hereinafter) while an aqueous solution of hydroxypropyl cellulose (HPC-SSL, manufactured by Nippon Soda Co. Ltd., the same applies hereinafter) (5% by weight) were sprayed, whereby core parts comprising Compound (A) were obtained. Then, powder coating of the resulting core part with 480 g of sucrose powder was carried out while an aqueous solution of hydroxypropyl cellulose (5% by weight) was sprayed in the same manner, whereby intermediate granules were obtained.
Granules having film: In accordance with the formulation shown in Table 1, the components of a film were dissolved and dispersed in purified water, whereby a spray liquid with a solid content of 11.6% by weight was prepared. Onto 800 g of the intermediate granules obtained above, the spray liquid was sprayed using CF-360 until a film to have 17.4 parts by weight in the dry state relative to 100 parts by weight of the intermediate granules, whereby granules were obtained.

### Example 2

Granules shown in Table 1 were produced in accordance with the following procedure.
Intermediate granules: A powder mixture of 6 g of Compound (A) and 294 g of sucrose powder were dispersed onto 600 g of purified sucrose spherical granules as a lead part using CF-360 while an aqueous solution of hydroxypropyl cellulose (5% by weight) was sprayed, whereby core parts comprising Compound (A) were obtained. Then, powder coating of the resulting core part with 300 g of sucrose powder was carried out while an aqueous solution of hydroxypropyl cellulose (5% by weight) was sprayed in the same manner, whereby intermediate granules were obtained.
Granules having film: granules were obtained in the same manner as in Example 1.

### Example 3

Granules shown in Table 1 were produced in accordance with the following procedure.
Intermediate granules: A powder mixture of 6 g of Compound (A), 78 g of sucrose powder and 36 g of corn starch (Starch 1500, manufactured by Colorcon Co. Ltd., the same applies hereinafter) were dispersed onto 600 g of purified sucrose spherical granules as a lead part using CF-360 while an aqueous solution of hydroxypropyl cellulose (5% by weight) was sprayed, whereby core parts comprising Compound (A) were obtained. Then, powder coating of the resulting core part with a powder mixture of 336 g of sucrose powder and 144 g of corn starch was carried out while an aqueous solution of hydroxypropyl cellulose (5% by weight) was sprayed in the same manner, whereby intermediate granules were obtained.
Granules having film: granules were obtained in the same manner as in Example 1.

### Example 4

Granules shown in Table 1 were produced in accordance with the following procedure.
Intermediate granules: A powder mixture of 6 g of Compound (A), 113 g of sucrose powder and 1 g of light anhydrous silicic acid (Sanlysia 350, manufactured by Fuji Silysia Chemical Ltd.) were dispersed onto 600 g of purified sucrose spherical granules as a lead part using CF-360 while an aqueous solution of hydroxypropyl cellulose (5% by weight) was sprayed, whereby core parts comprising Compound (A) were obtained. Then, powder coating of the resulting core part with a powder mixture of 475 g of sucrose powder and 5 g of light anhydrous silicic acid was carried out while an aqueous solution of hydroxypropyl cellulose (5% by weight) was sprayed in the same manner, whereby intermediate granules were obtained.
Granules having film: granules were obtained in the same manner as in Example 1.

### Example 5

Granules shown in Table 1 were produced in accordance with the following procedure.
Intermediate granules: A powder mixture of 6 g of Compound (A), 108 g of sucrose powder and 6 g of talc (Lissbran, manufactured by Kihara Kasei Co. Ltd., the same applies hereinafter) were dispersed onto 600 g of purified sucrose spherical granules as a lead part using CF-360 while an aqueous solution of hydroxypropyl cellulose (5% by weight) was sprayed, whereby core parts comprising Compound (A) were obtained. Then, powder coating of the resulting core part with a powder mixture of 456 g of sucrose powder and 24 g of talc was carried out while an aqueous solution of hydroxypropyl cellulose (5% by weight) was sprayed in the same manner, whereby intermediate granules were obtained.
Granules having film: granules were obtained in the same manner as in Example 1.

### Comparative Example 1

Granules shown in Table 1 were produced in accordance with the following procedure.
Intermediate granules: Powder coating of 1080 g of purified sucrose spherical granules as a core part with a powder mixture of 6 g of Compound (A) and 114 g of sucrose powder was carried out using CF-360 while an aqueous solution of hydroxypropyl cellulose (5% by weight) was sprayed, whereby intermediate granules were obtained.
Granules having film: granules were obtained in the same manner as in Example 1.

### Comparative Example 2

Granules shown in Table 1 were produced in accordance with the following procedure.
Intermediate granules: Powder coating of 900 g of purified sucrose spherical granules as a core part with a powder mixture of 6 g of Compound (A) and 294 g of sucrose powder was carried out using CF-360 while an aqueous solution of hydroxypropyl cellulose (5% by weight) was sprayed, whereby intermediate granules were obtained.
Granules having film: granules were obtained in the same manner as in Example 1.

### Comparative Example 3

Granules shown in Table 1 were produced in accordance with the following procedure.
Intermediate granules: Powder coating of 600 g of purified sucrose spherical granules as a core part with a powder mixture of 6 g of Compound (A) and 594 g of sucrose powder was carried out using CF-360 while an aqueous solution of hydroxypropyl cellulose (5% by weight) was sprayed, whereby intermediate granules were obtained.
Granules having a film: granules were obtained in the same manner as in Example 1.

[Table 1]

**Table 1**

| | | | Example 1 | Example 2 | Example 3 | Example 4 | Example 5 | Comparative Example 1 | Comparative Example 2 | Comparative Example 3 |
|---|---|---|---|---|---|---|---|---|---|---|
| Intermediate granules | Core part | Nonpareil | 49.8 | 49.8 | 49.6 | 49.8 | 49.8 | 89.9 | 74.9 | 49.9 |
| | | Compound A | 0.5 | 0.5 | 0.5 | 0.5 | 0.5 | 0.5 | 0.5 | 0.5 |
| | | Sucrose | 9.5 | 24.4 | 6.5 | 9.4 | 9.0 | 9.5 | 24.5 | 49.4 |
| | | Com starch | | | 3.0 | | | | | |
| | | Light anhydrous silicic acid | | | | 0.1 | | | | |
| | | Talc | | | | | 0.5 | | | |
| | | Subtotal | 10.0 | 24.9 | 10.0 | 10.0 | 10.0 | 10.0 | 25.0 | 49.9 |
| | Core-coating layer | Sucrose | 39.8 | 24.9 | 27.8 | 39.5 | 37.9 | | | |
| | | Com starch | | | 11.9 | | | | | |
| | | Light anhydrous silicic acid | | | | 0.4 | | | | |
| | | Talc | | | | | 2.0 | | | |
| | | Subtotal | 39.8 | 24.9 | 39.7 | 39.9 | 39.9 | | | |
| | Hydroxypropyl cellulose (Total amount in core part and core-coating layer) | | 0.4 | 0.4 | 0.7 | 0.4 | 0.4 | 0.1 | 0.2 | 0.2 |
| | Subtotal | | 100.0 | 100.0 | 100.0 | 100.1 | 100.1 | 100.0 | 100.1 | 100.0 |
| Film | | Hydroxypropylmethyl-cellulose | 6.00 | 6.00 | 6.00 | 6.00 | 6.00 | 6.00 | 6.00 | 6.00 |
| | | Lactose | 3.30 | 3.30 | 3.30 | 3.30 | 3.30 | 3.30 | 3.30 | 3.30 |
| | | polyethyleneglycol | 1.20 | 1.20 | 1.20 | 1.20 | 1.20 | 1.20 | 1.20 | 1.20 |
| | | Triacetine | 0.90 | 0.90 | 0.90 | 0.90 | 0.90 | 0.90 | 0.90 | 0.90 |
| | | Titanium oxide | 3.49 | 3.49 | 3.49 | 3.49 | 3.49 | 3.49 | 3.49 | 3.49 |
| | | Yellow ferric oxide (yellow) | 0.08 | 0.08 | 0.08 | 0.08 | 0.08 | 0.08 | 0.08 | 0.08 |
| | | Red ferric oxide (red) | 0.04 | 0.04 | 0.04 | 0.04 | 0.04 | 0.04 | 0.04 | 0.04 |
| | | Talc | 2.40 | 2.40 | 2.40 | 2.40 | 2.40 | 2.40 | 2.40 | 2.40 |
| | Subtotal | | 17.41 | 17.41 | 17.41 | 17.41 | 17.41 | 17.41 | 17.41 | 17.41 |
| Total amount | | | 117.4 | 117.4 | 117.4 | 117.5 | 117.5 | 117.4 | 117.5 | 117.4 |
| (unit: weight ratio) | | | | | | | | | | |

### Test example 1 (Photostability test)

A photostability test was carried out using the respective granules obtained in Examples 1 to 5 and Comparative Examples 1 to 3. The photostability test was carried out by uniformly placing the respective granules on a dish and exposing the granules to light of 1,000 Lux of daylight white light (D65) lamp in a thermostat bath at 25°C and at 60% relative humidity for 50 days (1,200,000 Lux·hr). After the light exposure, sampling was carried out, and the generation amount of analogous substances of Compound (A) was obtained by high performance liquid chromatography. The results of the photostability test are shown in Table 2. An analogous substance (B) is a geometric isomer of Compound (A). It is generated by photoisomerization and is not generated in the solid products during storage under shading conditions.
Conditions for high performance liquid chromatography
Column: Inertsil C8 4.6 x 250 mm GL Sciences Inc.
Column temperature: Constant temperature at around 40°C
Mobile phase: 0.05 mol/L phosphate buffer (pH 3.5) :
acetonitrile = 550 mL : 450 mL + 2.3 g of sodium lauryl sulfate Detection method: UV absorptiometry (wavelength of 299 nm)

[Table 2]

**Table 2**

| | Example 1 | Example 2 | Example 3 | Example 4 | Example 5 | Comparative Example 1 | Comparative Example 2 | Comparative Example 3 |
|---|---|---|---|---|---|---|---|---|
| Generation amount of analogous substance (B) (%) | 0.00 | 0.00 | 0.00 | 0.00 | 0.00 | 0.13 | 0.09 | 0.03 |
| Total generation amount of analogous substances other than B (%) | 0.01 | 0.00 | 0.33 | 0.14 | 0.15 | 2.31 | 1.51 | 0.60 |

From Table 2, significant suppression of the generation of analogous substance (B) and other analogous substances under the light exposure was observed in the respective granules obtained in Examples 1 to 5, in which each of the intermediate products are composed of a core part and a core-coating layer compared with the respective granules obtained in Comparative Examples 1 to 3, which do not have a core-coating layer. That is, it was elucidated that the solid products which comprise intermediate products including a core part comprising an active substance, and a core-coating layer covering the core part and comprising a powder-coating agent; and a film covering the intermediate products and comprising a compound having a photocatalytic activity or a semiconducting property is solid products excellent in storage stability such as photostability.

### Example 6

Granules as shown in Table 3 were produced in accordance with the following procedure.
Intermediate granules: A powder mixture of 23.55 g of Compound (A), 368.8 g of sucrose powder [the above-mentioned sucrose was pulverized using a pulverizer (Sample Mill KIIWG-1F, manufactured by Fuji Paudal Co. Ltd.) thereby obtaining powder with a volume average particle size of about 30 µm, the same applies hereinafter] and 2.83 g of light anhydrous silicic acid (Adsolider 101, manufactured by Freund Industrial Co. Ltd., the same applies hereinafter) were dispersed onto 2000 g of purified sucrose spherical granules as a lead part using CF-360 while an aqueous solution of hydroxypropyl cellulose (5.9% by weight) was sprayed, whereby core parts comprising Compound (A) were obtained. Then, powder coating of the resulting core part with a powder mixture of 1587.8 g of sucrose powder and 12.25 g of light anhydrous silicic acid was carried out while an aqueous solution of hydroxypropyl cellulose (5.9% by weight) was sprayed in the same manner, whereby intermediate granules were obtained (the above-mentioned production of intermediate granules were repeated three times).
The resulting intermediate granules were dried using a vented dryer (VD-1000SJ, Nitto Rika Kogyo Co. Ltd.) and subjected to sieving (355 to 820 µm) using a vibration sieve 502CBH (manufactured by Fuji Paudal Co. Ltd.). The yield of the obtained intermediate granules were high (sieving yield of 96%).
Granules having film: In accordance with the formulation shown in Table 3, the components of a film were dissolved and dispersed in purified water, whereby a spray liquid with a solid content of 11.6% by weight was prepared. Spray coating of 9300 g of the intermediate granules obtained above with the spray liquid was carried out using a fluidized bed granulation dryer (Flow coater FLO-15EX, manufactured by Freund Industrial Co. Ltd.,) until a film to have 17.4 parts by weight in the dry state relative to 100 parts by weight of the intermediate granules, whereby granules were obtained.
The resulting coated granules were subjected to sieving (355 to 850 µm, sieving yield of 96%) using a vibration sieve 502CBH (manufactured by Fuji Paudal Co. Ltd.), and light anhydrous silicic acid was added and mixed in an amount of 0.1% part by weight per 99.9% parts by weight of the coated granules, whereby products was obtained.

[Table 3]

**Table 3**

| | | | Example 6 |
|---|---|---|---|
| Intermediate granules | Core part | Nonpareil | 49.9 |
| | | Compound A | 0.6 |
| | | Sucrose | 9.2 |
| | | Light anhydrous silicic acid | 0.1 |
| | | Subtotal | 9.9 |
| | Core-coating layer | Sucrose | 39.6 |
| | | Light anhydrous silicic acid | 0.3 |
| | | Subtotal | 39.9 |
| | Hydroxypropyl cellulose (Total amount in core part and core-coating layer) | | 0.3 |
| | Subtotal | | 100.0 |
| Film | | Hydroxypropylmethylcellulose | 6.00 |
| | | Lactose | 3.30 |
| | | polyethyleneglycol | 1.20 |
| | | Triacetine | 0.90 |
| | | Titanium oxide | 3.49 |
| | | Yellow ferric oxide (yellow) | 0.08 |
| | | Red ferric oxide (red) | 0.04 |
| | | Talc | 2.40 |
| | Subtotal | | 17.41 |
| Light anhydrous silicic acid | | | 1.20 |
| Total amount | | | 118.6 |

### Industrial Applicability

According to the present invention, solid products excellent in storage stability such as photostability and a method for producing the same are provided.

## Claims

1. Solid products comprising: intermediate products including a core part comprising an active substance, and a core-coating layer covering the core part and comprising a powder-coating agent; and a film covering the intermediate products and comprising a compound having a photocatalytic activity or a semiconducting property.

2. The solid products according to claim 1, wherein the compound having a photocatalytic activity or a semiconducting property is titanium oxide, iron oxide, zinc oxide or silicon oxide.

3. The solid products according to claim 1 or 2, wherein the active substance is an active substance having reactivity with free radical or a secondary substance generated by a reaction thereof.

4. The solid products according to any one of claims 1 to 3, wherein the powder-coating agent is one or more member(s) selected from sugars, starches, starch derivatives, celluloses and cellulose derivatives.

5. The solid products according to any one of claims 1 to 3, wherein the powder-coating agent is a combination of a sugar, a starch and/or a slightly water-soluble inorganic salt.

6. The solid products according to claim 4 or 5, wherein the sugar is lactose or sucrose.

7. The solid products according to any one of claims 1 to 6, wherein the form of the solid products are powders, fine granules or granules.

8. The solid products according to any one of claims 1 to 6, wherein the form of the solid products are tablets.

9. A method for producing the solid products according to any one of claims 1 to 8, which comprises the step of coating a core part comprising an active substance with constituent components of a core-coating layer comprising a powder-coating agent by a powder coating method.
